# EUROPEAN PATENT SPECIFICATION

(11) **EP 1 654 284 B1**
(45) Date of publication and mention of the grant of the patent: **15.04.2009**
(21) Application number: 04764046.1
(22) Date of filing: 12.08.2004
(51) Int. Cl.: C07K 14/81, A61L 2/00

(54) **PROCESS FOR PREPARING AN ALPHA-1-ANTITRYPSIN SOLUTION**
VERFAHREN ZUR HERSTELLUNG EINER ALPHA-1-ANTITRYPSINLÖSUNG
PROCEDE DE PREPARATION D'UNE SOLUTION D'ALPHA-1 ANTITRYPSINE

(30) Priority: 12.08.2003 US 494097 P
(43) Date of publication of application: 10.05.2006
(73) Proprietor: Octapharma AG, 8853 Lachen (CH)
(72) Inventor: SCHULZ, Petra, A-1100 Vienna (AT); RÖMISCH, Jürgen, A-2440 Gramatneusiedl (AT)
(74) Representative: von Kreisler Selting Werner
(86) International application number: PCT/EP2004/009043
(87) International publication number: WO 2005/014648

(56) References cited:
- EP-A- 0 288 841
- EP-A- 0 436 086
- EP-A- 0 525 502
- WO-A-94/26287
- WO-A-98/56821
- DE-C- 4 407 837
- US-A- 4 629 567

## Description

The invention relates to a process for preparing a solution containing alpha-1-antitrypsin (A1AT), and to an A1AT.

A1AT is a glycoprotein having a molecular weight of about 55,000 Dalton and belongs to the family of serine-protease inhibitors. A1AT can inhibit the activity of a number of proteases, for example, trypsin, on which the name of the inhibitor is based for historical reasons. Physiologically, elastase is a target protease which is both involved in especially tissue and matrix reconstruction and degradation processes and released by cells such as granulocytes and is thus involved in inflammatory processes. The duration of elastase activity is limited in time and space and regulated essentially by the inhibitor A1AT. The dysregulation of such an activity leads to the quick degradation of tissue and may have pathophysiological consequences. In addition, inflammatory processes are initiated and/or promoted. A known example of a reduced or lacking control of elastase is the progressing local tissue degradation in the lung with accompanying inflammatory phenomena, which progressively leads to an emphysema and is thus accompanied with an, in part significantly, limited lung function. In the final stage, this may lead to the patient's death, which can be prevented ultimately only by a lung transplantation.

Such patients suffer from lacking A1AT or A1AT limited in its function. The inhibitor is normally produced and secreted in relatively large amounts by the liver and circulates in the blood plasma in relatively high concentrations (a typical concentration is 1.3 mg/ml). In addition, physiologically effective and sufficient concentrations of A1AT are found in the organs, especially in the lung fluid (epithelial lining fluid), of healthy people. If this A1AT concentration is significantly reduced or if the A1AT present is limited in its function or inactive (inactivated), there is an uncontrolled degeneration of lung tissue with the above mentioned consequences.

The reasons for lacking A1AT or A1AT reduced in its inhibitory function are mainly genetic defects. The so-called "Z" mutation, especially in homozygous (PiZZ) individuals, results in polymerization of the A1AT molecules already in the cells synthesizing them. Accordingly, the A1AT can no longer reach the circulation, or only so in very small amounts. This results, on the one hand, in a lacking inhibitory activity, which is manifested especially in the lung over prolonged periods of time, and on the other hand, in an enrichment of the polymers in the liver cells and thus in corresponding functional disorders. Heterozygous PiZ individuals have a correspondingly reduced inhibitory potential. Further mutations with similar defects are known.

According to current estimations, the prevalence of the PiZZ mutation in the USA is on the order of 1/1600 of the population; accordingly, the number of carriers of the mutation is significantly higher, of which presumably only 10% have been identified.

Of the patients who suffer from lung functional disorders (progressive emphysema) based on A1AT deficiency or A1AT reduced in function, not all can be treated currently since A1AT for the treatment and/or prophylaxis is not sufficiently available as an approved medicament. An established treatment is based on the intravenous administration of A1AT-containing solutions prepared from donor plasmas. The established and currently recommended dosage is 60 mg of A1AT per kg of body weight per week, which corresponds to an average consumption of 16-20 grams of A1AT per patient per month. This in turn corresponds to a quantity which is contained on average in 15 liters of plasma. Considering that only part of the inhibitor contained in the starting material plasma is obtained in a pure form as a preparation, a multiple of the 15 I of normal plasma is required as a raw material for one patient per month. The total volume of plasma required as a raw material for recovering A1AT and thus for the permanent treatment of patients is correspondingly large.

Established preparation methods for producing A1AT preparations use the so-called Cohn IV1 paste as a starting material. The latter is prepared by means of the Cohn-Oncley method, which is familiar to the skilled person, or by a modification thereof, based on the fractional separation of plasma proteins with varying, essentially, the concentration of ethanol added, the pH value adjusted and the temperature of the solution. In addition to A1AT, the so-called fraction IV1 usually contains a wide variety of other plasma proteins, in part in amounts already reduced by previous precipitation steps. A variation of this process is the Kistler-Nitschmann method. Accordingly, a fraction which is similar to the Cohn IV1 fraction as well as other A1AT-containing fractions, such as the so-called supernatant I+II+III, may also be used as a starting material.

Various methods for preparing a more or less pure A1AT preparation have been described. The use ion-exchange chromatography for the enrichment of A1AT, especially by means of anion-exchangers, has been reported repeatedly (Gray et al., 1960; Crawford et al., 1973; Chan et al., 1973; etc.). However, this preparation step alone does not yield an A1AT preparation having a purity which corresponds to the state of the art. Therefore, other preparation steps are employed, in part in combination with ion-exchangers. For example, adsorption or precipitation methods are used, such as incubation with polyethylene glycol (US-A-4,379,087), with zinc chelate or heparin adsorbents (US-A-4,629,567) or others. These methods are used for the (further) purification of A1AT, but in each of them a more or less large loss of product yield must be put up with. In principle, the product loss increases as the number of preparation steps increases. In addition, this is often accompanied with an extension of preparation time, which both may detract from the integrity and activity of A1AT and increases the production cost.

In addition to the protein preparation steps, so-called virus inactivation steps or depletion steps are an essential part of preparation processes for protein products prepared from plasma. In addition to the so-called SD (solvent/detergent) method, which inactivates corresponding viruses by damaging their protecting lipid envelope, thermal inactivation methods, for example, pasteurization (heat treatment for 10 hours at 60 °C), are applied for increasing virus safety. Filtration through "nanofilters" retains viruses, whether they are lipid-enveloped of lipid-free, usually depending on the size of the viruses. It is state of the art to integrate two process steps each of which is effective by itself and based on different principles together in one production process for maximum virus safety.

Depending on the protein, stabilizers, for example, amino acids or sugars, are added during these process steps for stabilizing the protein. Accordingly, these must be removed from the A1AT-containing solution later. In the SD method, detergents are added as active agents for virus inactivation which must be removed in the further course of the preparation process by suitable methods. For this purpose, adsorption to hydrophobic matrices, such as chromatography with immobilized C18 chains, has become established. Such chromatography is again accompanied with product yield losses and included the above mentioned drawbacks of each (further) chromatographic step in a method. In addition, these matrices are used repeatedly as a rule, i.e., cost-intensive and time-consuming matrix regeneration steps are required.

Accordingly, an alternative, effective and quick method for the qualitative removal of detergents which dispenses with a chromatographic step has been described (WO 94/26287, US-A-5,817,765). Thus, the detergent-containing protein solution is brought to superphysiological concentrations (≥ 0.5 M) of a salt, for example, Na citrate, to form detergent-containing particles which can be separated off simply by filtration, for example. In the following, this method is referred to as "detergent/salting-out" method.

In the examples of WO 94/26287, the "detergent/salting-out"-method is applied to three isolated proteins in solution, which are transferrin, antithrombin III and albumin. In the examples, the process leads to a recovery of protein activity of 95%, respectively, and to the reduction of the detergent concentration. If the method is applied under conditions, such that the yield of the target protein is not affected too much, frequently the concentration of Triton in the product is still high. In example 4 of WO 94/26287, the inventors are able to recover 95% of albumin activity, but obtain a product comprising 250 ppm Triton X-100 and 35 ppm TNBP. Especially when producing medical preparations, Triton X-100 concentrations above 50 ppm, preferably above 10 ppm should be avoided, and it is generally desirable to reduce the detergent contents as much as possible.

The object of the invention was to provide a process for purifying an A1AT preparation which results in a high purity and safety product as effectively and quickly as possible. Preferably, during the process the activity and/or the quality of A1AT should not be affected negatively and the detergents should be removed to levels which are acceptable for medical preparations.

It was another object of the invention to provide a method for the purification of solutions comprising A1AT, during which other protein components are removed. Preferably, the A1AT solution should also be depleted of other components such as lipids or viruses.

The object is achieved by a process for purifying A1AT from A1AT-containing solutions, from other protein components comprising the steps:
(a) subjecting an A1AT-containing solution to ion-exchange chromatography;
(b) adding detergents and optionally a solvent for inactivating lipid-enveloped viruses;
(c) followed by increasing the salt concentration to salt out the detergents.

Furthermore, the problem of the invention is solved by the embodiments as defined by claims 1 to 11. The process for purifying A1AT from A1AT-containing solutions from other protein components for example, from the reconstituted plasma fraction IV1 (Cohn), in principle consists of only two very efficient process steps in terms of its enrichment of A1AT: namely chromatography by means of an anion-exchanger, and SD virus inactivation treatment with Triton X-100 and TnBP, followed by salting out the virus inactivation agents.

It has been found that the last mentioned step can also be used very effectively in A1AT-containing solutions.

The process of the invention is especially useful when the A1AT comprising solution comprises significant amounts proteins different from A1AT. Surprisingly, under the process conditions, this process step not only can be used for removing detergents for virus inactivation, but additionally has a significant purification effect with respect to the separation of any protein, lipoprotein and lipid impurities present without adversely affecting the yield of A1AT. In combination with the above mentioned anion-exchange chromatography, essentially an A1AT product is obtained which has a purity of > 90%, preferably > 95% and contains A1AT in its active form. The process step of solvent detergent treatment and salting-out recovers active A1AT at a yield of ≥ 80 %. If reconstituted paste IV1 is used as the starting material for instance, the process of the invention allows the purification of A1AT due to removal of α-2-macroglobulin, haptoglobin, α-1 acidic glycoprotein, IgG, IgA and IgM from solutions comprising A1AT. In specific embodiments of the invention, the A1AT-comprising solution after the salting-out step recovers < 10% of α-2-macroglobulin, < 40% of haptoglobin, < 10% α-1 acidic glycoprotein, < 10% IgG, < 10% IgA and/or < 10% IgM, referring to the A1AT solution prior to the Solvent/detergent treatment, which is for instance the eluate of a prior anion-exchange chromatography. In preferred embodiments of the invention, the initial A1AT comprising solution comprises up to 50, up to 20 or up to 10 % (w/w) of other proteins. The initial solution preferably comprises at least 1, 2 or 5 % (w/w) of other proteins.

Even further, it was also observed that surprisingly the process of the invention is also useful as a virus inactivation and/or depletion step. Even though A1AT is recovered in high yields, the product is not only depleted or reduced from other protein components but also from viruses. This is especially useful for non-lipid-coated viruses, because the lipid coated viruses will already be inactivated by the detergent treatment. The process of the invention is thus also a process for the virus-inactivation, especially of non-lipid coated viruses, in solutions comprising A1AT.

The finding that a "detergent/salting-out" method as described in WO 94/26287 is applicable in the present case, leads to a removal of detergent to levels acceptable in medical preparations, and furthermore results in a highly specific enrichment of A1AT, is surprising. One would assume that the method of WO 94/26287 would result in products which have reduced detergent concentrations, which nonetheless might still be too high for pharmaceutical products, and in which all proteins and other components except the detergents are recovered at similar rates. Therefore one would not assume that the process of WO 94/26287 would be useful in the purification of a protein from other proteins.

The use of hydrophobic (interaction) chromatography (HIC), for example, on Phenyl-Sepharose^{®}, is desirable if an A1AT product having a still higher degree of purity is to be achieved. The performance of this step suggests itself, in particular, subsequently to detergent/salting-out treatment, since the protein binding to a hydrophobic matrix or ligands is usually mediated in the presence of superphysiological salt concentration. The elution of bound proteins is then effected, for example, by reducing the salt concentrations. Correspondingly, after the removal of the "salted-out" detergent directly of after a reasonable decrease of the salt concentration (by dilution or ultrafiltration/diafiltration; UF/DF), the A1AT-containing solution can be contacted with the hydrophobic matrix and the chromatography performed as familiar to the skilled person.

In addition to the SD treatment of the A1AT-containing solution, at least one further step for virus inactivation, virus removal and/or prion removal can be integrated in the process, for example, a thermal virus inactivation of the A1AT-containing solution. These steps can be performed, in particular, in solution directly following the process step of salting-out since the amounts of salts contained in the solution, especially sodium citrate, serve as stabilizers during the thermal treatment. As another possibility, the thermal treatment of the lyophilized product suggests itself. Alternatively or in addition, any suitable filtration method for the removal of viruses or prions may be included. Especially nanofiltration is familiar to the skilled person and may be integrated in the process preferably with commercially available filters having pore sizes within a range of from 15 to 20 nm or any suitable filtration to remove viruses and/or prions. The virus removal might be improved by the addition of amino acids, preferably at a concentration of 0,1 M for each amino acid. In a preferred embodiment glycine is added at a concentration above 0,2 M. A preferred method is described by Yokoyama et al. (2004, Vox Sanguinis 86, 225-229).

Generally suitable are A1AT-containing solutions obtained from plasma and its fractions or recombinant or transgenically expressed A1AT.

In a preferred embodiment of the process, the paste IV1 (Cohn) is reconstituted with water or a buffered solution, more preferably with a 20 mM Tris-buffered solution to reach a ratio of solution to paste of ≥ 3:1 (preferably > 10:1), subsequently contacted with an ion-exchange gel, preferably an anion-exchange gel, in a preferred embodiment a DEAE Sepharose^{®} (Amersham), more preferably DEAE-Sepharose^{®} Fast Flow, the gel is washed, and A1AT is eluted by increasing the ion strength. Virus inactivation is effected, for example, by the method according to EP-A-0 131 740. The optional addition of stabilizing agents is followed by the addition of virus-inactivating agents, preferably Triton X-100, Polysorbate 80 (Tween 80), TnBP and/or caprylic acid/caprylate, preferably to final concentrations of ≥ 0.1% (w/w) Triton and Tween 80, ≥ 0.03% (w/w) TnBP, ≥ 0.1 mM caprylic acid/caprylate. After an appropriate incubation time, preferably ≥ 0.1 hours, more preferably ≥ 1 hour, at ≥ 4 °C, more preferably at ≥ 15 °C, the salt concentration is increased, especially to a concentration of ≥ 0.5 M, especially with citrate. Particles formed thereby are removed, especially by filtration. Rewashing the filters or the separated particles can lead to an increase of A1AT yield. This may be followed by a further virus-inactivation step, preferably pasteurization in the presence of ≥ 0.5 M sodium citrate, amino acids, sugars or mixtures of these substances. The subsequent lowering of the concentration of the added substances is preferably effected by ultra-/diafiltration. More preferably, the subsequent separation of virus particles is effected by means of nanofilters, preferably by means of filters having pore sizes of 15-20 nm. The A1AT thus obtained can be stored as a liquid or frozen preparation, or freeze-dried by methods familiar to the skilled person.

The A1AT product thus obtained can be administered as a solution subcutaneously, intramuscularly, topically or as an aerosol, preferably intravenously. As a dried material, it may also be used for inhalation in a powder form. Application in admixture with other solutions, for example, for intravenous application, is possible. A possible dosage is, for example, 60 mg/kg of body weight per week, or 250 mg/kg per month.

Another preferred process includes HIC in addition to the process steps mentioned above, preferably HIC by means of a phenyl matrix, preferably after SD treatment and the salting-out of virucidal agents and other impurities, as set forth above. Preferably, a negative purification is performed , i.e., the valuable substance A1AT passes the chromatographic matrix unbound, and undesirable substances are bound and thus removed from the process solution.

In another preferred process which may include the above mentioned HIC, chromatography on immobilized heparin, preferably by means of heparin-sepharose or heparin-fractogel, is performed. Thus, the A1AT-containing solution is contacted with the heparin gel in a column or in a batch process. Enriched A1AT passes the column unbound, or is found in the supernatant after gel separation. This process step is preferably performed before or after the above mentioned ion-exchange chromatography, or after the salting-out of the detergent and reducing the ion strength of the solution, for example, by dialysis.

The process according to the invention is further illustrated by the following Example:

### Example 1:

For reconstitution, frozen Cohn IV1 paste is dissolved in 20 mM Tris solution at a weight ratio of 1:9 with stirring for 3 hours at alkaline pH.

### Anion exchange chromatography

A preferred chromatographic material for this process step is DEAE-Sepharose FF (Fast Flow), although the conditions may also be adapted by the skilled person for each anion-exchange material. A packed DEAE-Sepharose FF chromatographic column is equilibrated with a 20 mM Tris solution (pH 8.0). Thereafter, dissolved Cohn IV1 paste is applied at pH 8.0. Washing is effected with an equilibration buffer, followed by a washing step with a buffer solution. The A1AT bound to the matrix can then be eluted by washing the column with 20 mM Tris, .0.075 M NaCl, pH 8.0. The specific activity of the thus obtained A1AT solution is about 0.5 PEU/mg of protein (PEU: plasma equivalent unit; corresponds to the amount or activity of A1AT which is found on average in one milliliter of human plasma). The column is eluted with a high salt buffer (e.g., 2 M NaCl), and the chromatographic gel can subsequently be regenerated by per se known methods.

### Solvent/detergent treatment

The thus obtained eluate is concentrated by ultrafiltration. Subsequently, a premixed solution of Triton X-100, TnBP and water for pharmaceutical use (WFI) is added to reach a final concentration of 1% (w/w) Triton X-100 and 0.3% (w/w) TnBP. The SD treatment is performed for 4 hours at 20 °C with mild stirring.

### Salting-out

For removing the SD reagents and precipitating undesirable accompanying proteins, the A1AT-containing solution is diluted with a solution which contains 1.5 M sodium citrate and 20 mM Tris at pH 7.0. The addition to a citrate concentration of 1 M is effected over at least 15 minutes with stirring. Subsequently, the process solution is incubated for at least one hour with mild stirring. The whitish precipitate formed is subsequently separated off by filtration. This decreases the concentration of SD reagents to below 10 ppm, and undesirable accompanying proteins and denatured A1AT is also separated off.

After this production step, the specific activity of A1AT is at least 0.8 PEU/mg (PEU: plasma equivalent unit; corresponds to the amount or activity of A1AT which is found on average in one milliliter of human plasma).

### Nanofiltration

After the removal of low-molecular substances by means of UF/DF, the solution thus obtained is filtered through filters with a nominal exclusion size of 15-20 nm, such as DV20 filters of the company Pall, in order to further increase the virus safety of the A1AT product.

### Results:

The concentrations of Triton X-100 and TNBP in the product after filtration were below 5 ppm. The amount of A1AT and other protein components in the product was determined and compared to the amounts determined before the solvent/detergens-treatment. The following recoveries were calculated:

| | |
|---|---|
| A1AT | > 80% |
| α-2-macroglobulin | < 10% |
| haptoglobin | < 40% |
| α-1 acidic glycoprotein | < 10% |
| IgG | < 10% |
| IgA | < 10% |
| IgM | < 10% |

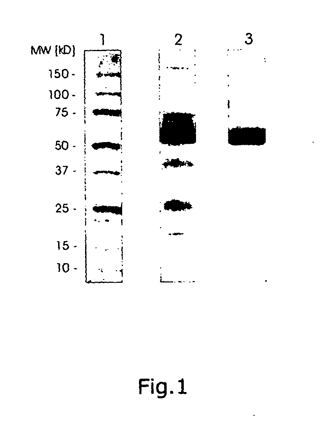

The results show that the in the product A1AT was recovered specifically in high amounts, whilst the product was depleted significantly from other protein components. This is further illustrated by figure 1, which shows the result of an SDS-PAGE of the solution before the solvent/detergens treatment (lane 2) and after the salting-out step (lane 3). The A1AT corresponds to the broad band slightly above 50kD (in comparison to the molecular weight marker in lane 1). The various other protein components clearly detectable in the starting solution are reduced significantly.

### Example 2

Modifying the process as described in Example 1, the A1AT-containing eluate from the ion-exchange chromatography is contacted with heparin-sepharose. The A1AT passes the heparin-sepharose column without being bound. If batch operation is employed, the A1AT remains in the supernatant. The further processing of the column eluate or of the supernatant from the batch variant is effected as described in Example 1. The thus obtained product is characterized by increased purity.

**Figure 1** shows the result of an SDS-PAGE under reducing conditions, gradient 4-20%, Coomassie staining, 5 µg protein/lane. Lane 1: Molecular weight marker; lane 2: probe of a solution according to example 1 before the solvens/detergent-treatment; lane 3: probe of a solution according to example 1 after the salting-out step.

## Claims

1. A process for purifying from other protein components A1AT in A1AT-containing solutions, comprising the following steps:
(a) subjecting an A1AT-containing solution to ion-exchange chromatography;
(b) adding detergents and optionally a solvent for inactivating lipid-enveloped viruses;
(c) followed by increasing the salt concentration to salt out the detergents.

2. The process according to claim 1, wherein said A1AT-containing solution has been obtained from blood plasma or its fractions, preferably from a reconstituted plasma fraction IV1 (Cohn), or is derived from a recombinantly or transgenically expressed A1AT preparation or a fermentation supernatant.

3. The process according to claim 1 and/or 2, wherein ion-exchange chromatography is performed on an anion-exchange gel, preferably DEAE-Sepharose^{®} or DEAE-Sepharose^{®} Fast Flow.

4. The process according to any of claims 1 to 3, wherein said virus inactivation according to step (b) is effected with Triton X-100, Polysorbate 80 (Tween 80), TnBP and/or caprylic acid or caprylate, preferably at final concentrations of ≥ 0.1% (w/w) Triton and Tween 80, ≥ 0.03% (w/w) TnBP, ≥ 0.1 mM caprylic acid or caprylate, with an incubation time of ≥ 0.1 hours, preferably ≥ 1 hour, at ≥ 4 °C, especially at ≥ 15 °C.

5. The process according to any of claims 1 to 4, wherein the salt concentration of the solution is brought to ≥ 0.5 M in step (c) and particles formed thereby are preferably removed by filtration.

6. The process according to any of claims 1 to 5, wherein chromatography on hydrophobic chromatographic materials is performed.

7. The process according to any of claims 1 to 6, wherein a treatment of the A1AT-containing fraction with a material which contains heparin in an immobilized form (heparin gel) is performed.

8. The process according to any of claims 5 to 7, wherein a further virus inactivation step is performed afterwards, preferably pasteurization in the presence of ≥ 0.5 M sodium citrate, amino acids, sugars or mixtures thereof.

9. The process according to any of claims 1 to 8, wherein the ion strength of the solution is preferably reduced by ultra-/diafiltration.

10. The process according to any of claims 1 to 9, wherein a separation of virus particles is performed, preferrably by nanofiltration, preferably with filters having pore sizes of 15-20 nm.

11. The process according to any of claims 1 to 10, wherein the A1AT fraction obtained is stored as a liquid, frozen or freeze-dried preparation.

## Patentansprüche

1. Verfahren zum Reinigen von A1AT bezüglich anderer Proteinkomponenten in A1AT-haltigen Lösungen, die folgenden Schritte umfassend:
(a) Durchführen einer Ionenaustauschchromatographie mit einer A1AT-haltigen Lösung;
(b) Hinzufügen von Tensiden und gegebenenfalls eines Lösungsmittels zum Inaktivieren von lipidumhüllten Viren;
(c) anschließend Erhöhen der Salzkonzentration zum Aussalzen der Tenside.

2. Verfahren gemäß Anspruch 1, wobei die A1AT-haltige Lösung aus Blutplasma oder seinen Fraktionen, vorzugsweise aus einer rekonstituierten Plasmafraktion IV1 (Cohn), erhalten wurde oder aus einem rekombinant oder transgen exprimierten A1AT-Präparat oder einem Fermentierungsüberstand stammt.

3. Verfahren gemäß Anspruch 1 und/oder 2, wobei die Ionenaustauschchromatographie an einem Anionenaustauschergel, vorzugsweise DEAE-Sepharose^{®} oder DEAE-Sepharose^{®} Fast Flow, durchgeführt wird.

4. Verfahren gemäß einem der Ansprüche 1 bis 3, wobei die Virusinaktivierung gemäß Schritt (b) mit Triton X-100, Polysorbat 80 (Tween 80), TnBP und/oder Caprylsäure oder Caprylat, vorzugsweise in Endkonzentrationen von ≥ 0,1 Gew.-% Triton und Tween 80, ≥ 0,03 Gew.-% TnBP, ≥ 0,1 mM Caprylsäure oder Caprylat, mit einer Inkubationszeit von ≥ 0,1 Stunde, vorzugsweise ≥ 1 Stunde, bei ≥ 4°C, insbesondere bei ≥ 15 °C, durchgeführt wird.

5. Verfahren gemäß einem der Ansprüche 1 bis 4, wobei die Salzkonzentration der Lösung in Schritt (c) auf ≥ 0,5 M gebracht wird und die **dadurch** entstehenden Teilchen vorzugsweise durch Filtration entfernt werden.

6. Verfahren gemäß einem der Ansprüche 1 bis 5, wobei eine Chromatographie an hydrophoben chromatographischen Materialien durchgeführt wird.

7. Verfahren gemäß einem der Ansprüche 1 bis 6, wobei eine Behandlung der A1AT-haltigen Fraktion mit einem Material, das Heparin in immobilisierter Form enthält (Heparin-Gel), durchgeführt wird.

8. Verfahren gemäß einem der Ansprüche 5 bis 7, wobei danach ein weiterer Virusinaktivierungsschritt durchgeführt wird, vorzugsweise eine Pasteurisierung in Gegenwart von ≥ 0,5 M Natriumcitrat, Aminosäuren, Zuckern oder Gemischen davon.

9. Verfahren gemäß einem der Ansprüche 1 bis 8, wobei die Ionenstärke der Lösung vorzugsweise durch Ultra-/Diafiltration reduziert wird.

10. Verfahren gemäß einem der Ansprüche 1 bis 9, wobei eine Abtrennung von Viruspartikeln durchgeführt wird, vorzugsweise durch Nanofiltration, vorzugsweise mit Filtern mit Porengrößen von 15-20 nm.

11. Verfahren gemäß einem der Ansprüche 1 bis 10, wobei die erhaltene A1AT-Fraktion als flüssiges, gefrorenes oder gefriergetrocknetes Präparat aufbewahrt wird.

## Revendications

1. Procédé de purification, d'avec d'autres composants protéiniques, d'A1AT dans des solutions contenant de l'A1AT, comprenant les étapes suivantes :
(a) soumettre une solution contenant de l'A1AT à une chromatographie par échange d'ions ;
(b) ajouter des détergents et éventuellement un solvant pour inactiver les virus à enveloppe lipidique ;
(c) puis augmenter la concentration en sels pour relarguer les détergents.

2. Procédé selon la revendication 1, dans lequel ladite solution contenant de l'A1AT a été obtenue à partir de plasma sanguin ou de fractions de celui-ci, de préférence à partir d'une fraction de plasma reconstitué IV1 (Cohn), ou est dérivée d'un surnageant de fermentation ou d'une préparation d'A1AT exprimée par recombinaison ou par voie transgénique.

3. Procédé selon la revendication 1 et/ou 2, dans lequel la chromatographie par échange d'ions est réalisée sur un gel d'échange d'anions, de préférence DEAE-Sepharose^{®} ou DEAE-Sepharose^{®} Fast Flow.

4. Procédé selon l'une quelconque des revendications 1 à 3, dans lequel ladite inactivation de virus selon l'étape (b) est réalisée avec du Triton X-100, du Polysorbate 80 (Tween 80), du TnBP et/ou de l'acide caprylique ou du caprylate, de préférence à des concentrations finales supérieures ou égales à 0,1% (p/p) de Triton et de Tween 80, supérieures ou égales à 0,03% (p/p) de TnBP, supérieures ou égales à 0,1 mM d'acide caprylique ou de caprylate, avec une durée d'incubation supérieure ou égale à 0,1 heure, de préférence supérieure ou égale à 1 heure, à une température supérieure ou égale à 4°C, en particulier supérieure ou égale à 15°C.

5. Procédé selon l'une quelconque des revendications 1 à 4, dans lequel la concentration en sel de la solution est amenée à ≥ 0,5 M à l'étape (c) et les particules ainsi formées sont de préférence éliminées par filtration.

6. Procédé selon l'une quelconque des revendications 1 à 5, dans lequel une chromatographie est réalisée sur matières chromatographiques hydrophobes.

7. Procédé selon l'une quelconque des revendications 1 à 6, dans lequel un traitement de la fraction contenant de l'A1AT est réalisé avec une matière qui contient de l'héparine sous forme immobilisée (gel d'héparine).

8. Procédé selon l'une quelconque des revendications 5 à 7, dans lequel une autre étape d'inactivation de virus est ultérieurement réalisée, de préférence par pasteurisation en présence de ≥ 0,5 M de citrate de sodium, d'acides aminés, de sucres ou de mélanges de ceux-ci.

9. Procédé selon l'une quelconque des revendications 1 à 8, dans lequel la force ionique de la solution est de préférence réduite par ultra-/dia-filtration.

10. Procédé selon l'une quelconque des revendications 1 à 9, dans lequel une séparation des particules virales est réalisée, de préférence par nanofiltration, de préférence avec des filtres ayant une taille de pores de 15 à 20 nm.

11. Procédé selon l'une quelconque des revendications 1 à 10, dans lequel la fraction d'A1AT obtenue est conservée sous la forme d'une préparation liquide, congelée ou lyophilisée.
